# EUROPÄISCHE PATENTANMELDUNG

(11) **EP 3 733 156 A1**
(43) Veröffentlichungstag der Anmeldung: **04.11.2020**
(21) Anmeldenummer: 19172383.2
(22) Anmeldetag: 02.05.2019
(51) Int. Cl.: A61K 8/34, A61Q 19/00, A61K 31/05, A61K 36/185, A61K 9/00, A61P 29/00

(54) **C.B.D.M THERAPIE**

(71) Anmelder: Fóti, Gábor, 1162 Budapest (HU); Deme, Áron Barnabás, 1020 Wien (AT)
(72) Erfinder: Fóti, Gábor, 1162 Budapest (HU); Deme, Áron Barnabás, 1020 Wien (AT)

(57) **Zusammenfassung**

┌ eine Steigung der Versorgung von der Nährstoffaufnahme von Zellen
┌ das Nachlassen von Alterungsprozesse
┌ eine Zunahme von Leistungsfähigkeit der Muskulatur
┌ das Aufhören von Muskelmüdigkeit
┌ eine Verstärkung von Widerstandkapazität des Organismus

Öle, Creme, Pulver

Zu den meisten Massagearten braucht man ein bestimmtes Schmiermittel, damit unsere Hand diese auf den Körper des Patienten gleichmäßig verteilen kann. In den meisten Fällen werden verschiedene Öle verwendet, obwohl die unterschiedlichen alkoholenthaltende Flüssigkeiten, Creme oder Streupulver auch angemessen sind. Um den Körper ordentlich zu massieren, wird etwa fünfzig Millimeter Massageöl gebraucht.

Es kann bis einige Gelegenheiten dauern, solange man auf die passende Menge gewöhnt. Am Anfang kommt vor, dass manche zu viel Öl verwenden, wodurch kein passender Kontakt mit dem Empfänger geschaffen wird. Man braucht bloß eine dünne Schicht: Nur so viel, das die Haut nach der Massage völlig einzieht.

Bei der Erfindung dieses Produkt war unser Ziel, dass wir ein Produkt herstellen, welches ohne chemischen Inhalte und ohne Arzneimittel nur natürliche Substanzen beinhaltete, die oben erwähnten Problemen behandelt und löst bzw. ein wichtiger Bestandteil von physiotherapeutischen Behandlungen oder von Massagen sein kann.

Als wir an unserem Produkt arbeiteten, haben wir erkannt, dass die natürlichen Öle bzw. die Duftöle mit CBD und CBD beinhaltenden Produkte vermischt werden sollen um die Lösung für die oben genannten Krankheiten zu finden.

Der Vorteil dieses Produktes sei dass, die Lösung statt der Verwendung von Arzneimittel nur durch die regenerierende und gesunde Auswirkung von natürlichen CDB, oder CDB beinhaltenden Produkte vermischt mit Massageöle, Creme oder Pulver erreicht wird. Das Produkt muss im Laufe der Massage oder bei der psychotherapeutischen Behandlung in die Haut einmassieren. Aus diesem Grund wird es möglich dass, die positive Wirkung von CBD und von CBD beinhaltenden Produkte in die Oberfläche der Haut eindringen und effektiv sein können.

Patentierung

1. Ein heilendes Produkt mit einer natürlichen Basis welches CDB und CDB beinhaltenden Produkte mit Öle, Creme, Salben, Pulver vermischt.
2. Das CDB beinhaltende Produkt wird für Massage und für physiotherapeutische Behandlung gebraucht.

## Beschreibung

Der Gegenstand der Erfindung, ist ein heilendes Produkt mit einer natürlichen Basis, welches durch der Hautoberfläche verwendet, für die Milderung bzw. für die Beseitigung von Schmerzen im Bewegungsapparat sorgt.

Sie kann gegen chronischen Schmerzen, Krebserkrankungen, Crohn, Zuckerkrankheit, rheumatischen Arthritis, posttraumatischer Störung, Herz- und Gefäßsystemkrankheiten, Angststörungen, Antibiotikaresistenz Infektionen, Sklerose multiplex, Schizophrenie, Depression, Epilepsie, Schmerz, Gicht, Entzündung, Zuckerkrankheit, eingesetzt werden. Sie hilft noch bei Verringerung des Hungergefühls, bei der Gewichtsabnahme und bei der Schlaflosigkeit. Sie verfügt über eine antibakterielle, anti-diabetische, antiemetikische, antichemische, antisoriatische und neuroprotektive Wirkung. Das Produkt der Erfindung sind alle Öle, Salben, Cremes bzw. kosmetische Mitteln welche CBD beinhaltenden Pflanzen, Öle, Salben, Cremes oder Pulver beinhalten.

Der kannabidiol oder auch CBD genannt, ist einer nicht betäubenden Bestandteil der Cannabis Pflanze, welche ein riesiges therapeutisches Potenzial hat. Obwohl CBD keine psychoaktive Wirkung verursacht, wie das THC, hat in Kreisen der Wissenschaftler sowie bei Ärzten aber auch bei medizinischen Cannabis verwendeten Patienten - die CBD beinhaltenden Produkte für die Behandlung von Problemen wie chronische Schmerzen, Krebserkrankungen, Crohn, Zuckerkrankheit, rheumatische Arthritis, posttraumatische Störung, Herz- und Gefäßsystemkrankheiten, Angststörungen, Antibiotikaresistenz Infektionen, Sklerose multiplex, Schizophrenie, Depression, Epilepsie, Schmerz, Gicht, Entzündung, Zuckerkrankheit - für großes Interesse gesorgt.

Es werden weltweit zahlreiche wissenschaftliche Forschungen bezüglich der Wirkung CBD auf die oben genannten Krankheiten und auf andere Krankheiten geleistet. Die Wissenschaftler bezeichnen CBD als eine "abschweifende" Verbindung, da ihre therapeutische Wirkung auf verschiedene Weise offenbart wird.

Die umfassende preklinische Forschungen und einige klinische Überprüfungen haben gezeigt dass CBD ein starkes Antioxidationsmittel, und Antidepressiva sei, wobei sie verfügt über entzündungshemmende, antpsychotische, krebspreventive, neuroprotektive Eigenschaften. Kannabidiol ist im Stand die Genexpression zu modifizieren und die beta-amiloid Placks im Gehirn zu entfernen, welche für die Krankheit von Alzheimer zuständig ist

Das ist ein natürliches Molekül welches als kannabinoid oder als fitokannabinoid genannt wird. Dieses Molekül kommt am meisten in der Pflanze Cannabis (Haft) vor aber in kleineren Mengen ist es in Flachs sowie in Azaleen auch zu finden.

Die Wissenschaftler wissen zurzeit ungefähr von der Existenz von 100 kannabinoid. Neben THC ist CBD am meisten bekannt und erforscht. CBD im Vergleich zu THC hat keine psychoaktive Wirkung. Die oben genannten Krankheiten sind nur ein kleiner Bruchteil davon, welche Krankheiten mit CBD geheilt werden können. Es gibt aber noch viele Missverständnisse bezüglich der Wirkung von CBD und diese sollten nicht außer Acht gelassen werden, wenn man von dieser chemischen Zusammensetzung spricht.

Im Jahr 2011 bestätigt eine Überprüfung von der gegenwärtigen Medizinsicherheit (Current Drug Safety), die Tatsache, dass CBD auf die psychomotorischen und psychologischen Funktionen nicht störend wirkt.

Der Gegenstand der Erfindung, ist ein heilendes Produkt mit einer natürlichen Basis, welches durch der Hautoberfläche verwendet, für die Milderung bzw. für die Beseitigung von Schmerzen im Bewegungsapparat sorgt. Sie kann gegen chronischen Schmerzen, Krebserkrankungen, Crohn, Zuckerkrankheit, rheumatischen Arthritis, posttraumatischer Störung, Herz- und Gefäßsystemkrankheiten, Angststörungen, Antibiotikaresistenz Infektionen, Sklerose multiplex, Schizophrenie, Depression, Epilepsie, Schmerz, Gicht, Entzündung, Zuckerkrankheit eingesetzt werden. Sie hilft auch noch bei Verringerung des Hungergefühls, bei der Gewichtsabnahme und bei der Schlaflosigkeit. Sie verfügt über eine antibakterielle, anti-diabetische, antiemetikische, antichemische, antisoriatische und neuroprotektive Wirkung.

Die Massage ist ein mechanischer Stimulus auf der Oberfläche des Körpers, worauf die Antwortreaktion von dem Körper eine physische heilende Wirkung sei. Die Auswirkungen der Massage betreffen die menschlichen Organe entweder direkt oder indirekt, und haben beispielsweise auf den Muskelton und auf das Nervensystem eine positive Wirkung.

Stress bewirkt heutzutage immer mehr physische Beschwerden und wird als Grund für viele Krankheiten benannt. Es gibt viele Menschen die den Stress nicht behandeln können aber die Massage kann auch einem dabei helfen. Die hilft bei der richtigen Entspannung, und bei der Auflösung von Blockaden die Stress bewirken können. Die Massage erfrischt nicht nur die Haut sondern sie sorgt für die Erfrischung von Muskeln und Blutkreisumlauf und hat eine entspannende Wirkung auf das Nervensystem. Mit der Massage kann man die unwillkürliche Spannung der Muskeln auflösen und auf Funktion des Herzrhythmus wirken. Die Massage entspannt die Muskeln und beruhigt das Nervensystem, wodurch für eine ruhige, unbelastete Nacht gesorgt. Infolgedessen wird die Atmung gleichmäßig und der Blutdruck sinkt auch.

Die Massage reduziert direkt die Spannung des Muskels wodurch sich die Elastizität der Muskeln und die Leistungsfähigkeit verbessern, weshalb das Leeren von Abbauprodukten beschleunigt wird. Sie verfügt über eine allgemeine schmerzstillende Wirkung weil, während der Massage Histamin und Bradikinin im Organismus ausgeschüttet wird.

Die Massage breitet die Haargefäße des Körpers heraus, wodurch eine größere Aufnahme von Nährsubstanzen der Zellen ermöglicht wird, welche bei der Eliminierung der Toxine hilft. Sie entfernt die toten Oberhautzellen weshalb, die schnellere Erneuerung der neuen Hautzellen möglich wird. Der verstärkte Blutkreislauf sichert den passenden Feuchtigkeitsgehalt, deshalb kann die Haut straff, elastisch und schön bleiben.

Die Massage bewirkt:
┌ eine Linderung von Schmerzen
┌ eine Verstärkung von Kreislauf
┌ eine Beschleunigung von Stoffwechsel
┌ eine Verstärkung von lymphischen System
┌ eine Optimierung von Blutdruck und Puls
┌ eine gleichmäßige Atmung
┌ eine Erweiterung von Sauerstofferstattung
┌ eine Hypermia
┌ ein Wechsel von tote Oberhautzellen zu neuen Oberhautzellen
┌ eine Elastizität der Haut
┌ eine Straffheit der Haut

## Patentansprüche

1. Der Gegenstand der Erfindung gelangt entweder vor oder während der Massage bzw. während der physiotherapeutischen Behandlung durch die Haut in den Körper. Der Gegenstand der Erfindung ist für alle Massagenarten geeignet. Die Öle, die Salben, die Creme, die kosmetische Mittel können mit allen CBD beinhaltenden Pflanzen, Öle, Salbe, Creme bzw. Pulver vermischt werden.

2. Bei der Massage oder bei der physiotherapeutischen Behandlung wird der Gegenstand der Erfindung entweder mit der Hand oder mit einem dafür geeigneten Gerät auf die Oberfläche der Haut aufgetragen. Aus diesem Grund gelangt der CBD bzw. das CBD beinhaltende Produkt durch die Haut in den Körper.

3. Das Produkt der Erfindung ist die Vermischung von Öle, Salben, Creme oder kosmetische Mittel mit CBD beinhaltenden Pflanzen, Öle, Salben, Creme, oder Pulver.
Die Massage kann in jeder Körperhaltung und unter allen Umständen durchgeführt werden.

4. Bei der Massage oder bei der physiotherapeutischen Behandlung werden die Öle, Salben, Creme und kosmetische Mittel mit CBD beinhaltenden Pflanzen, Öle, Salben, Creme, Pulver bzw. als Rohstoff von CBD genannt, vermischt. Die Herstellung des Produktes wird auf unterschiedlichen Art und Weise erfolgen, wie zum Beispiel durch Schütteln, Destillieren, Pressung, oder durch Temperaturen mischen. Die Art und Weise hängt von der Stärke der Wirkung des Produktes ab.

5. Bei der Massage oder bei der physiotherapeutischen Behandlung können Produkte der Erfindung zwischen 0,01% bis 100% CBD Inhalt aufweisen.
